# EUROPEAN PATENT APPLICATION

(11) **EP 3 476 412 A1**
(43) Date of publication of application: **01.05.2019**
(21) Application number: 17198284.6
(22) Date of filing: 25.10.2017
(51) Int. Cl.: A61M 1/16, A61B 5/00, A61B 5/145, A61B 5/1455, G01N 21/05, G01N 21/71, G01N 21/85

(54) **A METHOD OF INLINE MEASURING A SUBSTANCE OF INTEREST IN BLOOD FLUID**

(71) Applicant: Nederlandse Organisatie voor toegepast- natuurwetenschappelijk onderzoek TNO, 2595 DA 's-Gravenhage (NL)
(72) Inventor: The designation of the inventor has not yet been filed
(74) Representative: V.O.

(57) **Abstract**

A method is disclosed for inline measuring a substance of interest in blood fluid. Blood fluid is circulated through a dialysis filter having a blood inlet and a blood outlet. A dialysate circuit comprises a dialysate inlet and dialysate outlet, thereby having the substance of interest permeating via the dialysis filter membrane to the dialysate circuit. A cuvette in the dialysate circuit is provided with a detector, for measuring a concentrate level of said substance of interest in said cuvette. In a first phase a first blood flow is provided through the dialysis filter, while providing a first dialysate flow in the dialysate circuit; in a second phase a second blood flow is provided through the dialysis filter, while providing a second dialysate flow in the dialysate circuit. A concentrate level of the substance interest in the blood fluid is derived from the measured concentrate level in the dialysate.

## Description

### FIELD

The present invention relates to a method and system for inline measuring a substance of interest in blood fluid.

### BACKGROUND

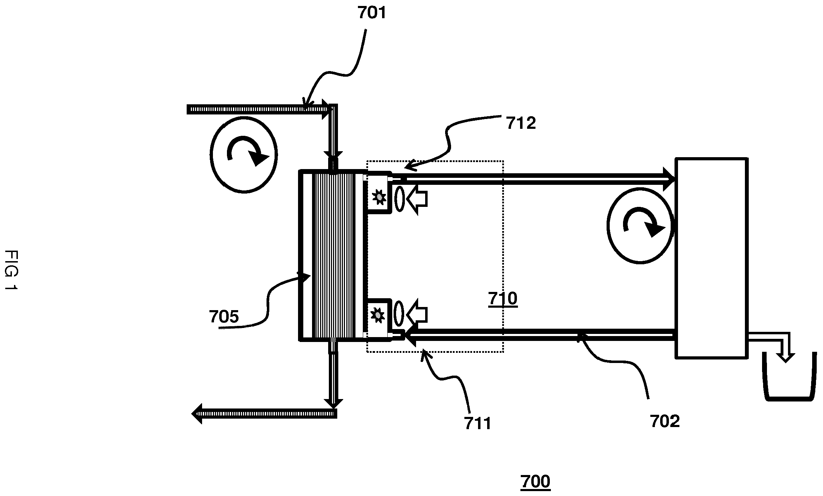

In fluid media environments, in particular, in fluids originating from the human body or fluids destined for insertion into the human body, such as dialysate, haemodiafiltration fluid or blood serum, but also in plant process environments, in the production of desalinated water etc, a desire exists to monitor the fluids' chemical composition or concentrations, in particular of electrolytes and other chemical traces. In the shown embodiment of Figure 1, part of a renal dialysis machine is shown which can e.g. be used for haemodialisys. In haemodialisys applications, patient's blood is lead through a dialysis filter, cleaned and fed back to the arterial circulation. The dialysis filter has e.g. a semipermeable membrane having on one side blood, and on the other side dialysate that cleans the blood. The dialysate may be used for a single pass and then be discarded, or it may be regenerated in a sorbent system for multipass. Especially when regenerating the dialysate, it is important to monitor and maintain a proper balance of electrolytes in the dialysate, in particular, of Na+, K+ and Ca2+. Too high Ca2+ induces risks of atherosclerosis, and too low may result in osteoporosis. Excessive Na+ may increase blood pressure, where low levels will result in low blood pressure. Also K+ is a critical electrolyte for which abnormal levels may lead to heart rythm problems (arrythmia) or even heart failure. Also such electrolyte monitoring is of importance for single pass dialysis systems. In WO2016007012 an in-line measurement or off-line analytical testing of fluid samples is proposed e.g. by compact pulsed lasers that combine high beam quality with high pulse energy. When carefully focused, such lasers can deliver energy densities that are strong enough to induce optical breakdown in liquids (in the order of 10xe10 W/cm²). In this way it is possible to generate a short lived plasma wherein the emission spectrum is indicative for the plasma composition.

For effective measurement of chemical concentrations in blood a difference measurement is proposed measuring a difference in chemical concentrations of diaysate flowing into and out of the blood filter.

Spectroscopy techniques of these kinds have been demonstrated in "Laser-induced breakdown spectroscopy (LIBS): "An overview of recent progress and future potential for biomedical applications", Rehse et al, Journal of Medical Engineering & Technology, 2012: 36(20;77-89).

In "An experimental and numerical study of the flow and mass transfer in a model of the wearable artificial kidney dialyzer" Rambod et al. (BioMedical Engineering OnLine 2010, 9:21), the effect of a pulsatile flow is discussed enhancing the efficiency of removing urea from blood. The reason for this phenomenon is attributed to a higher ultrafiltration rate caused by the higher convective mass transfer flux along the length of the fibers in the dialysis filter. Other state of the art techniques for measurement of chemical concentrations in blood include dedicated membranes that are ion-selective used in combination with electrodes. However, these membranes are not practical for inline measurements and suffer from pollution, ion-crosstalk, susceptibility for electromagnetic fields and drift. This still leaves a problem of measuring absolute concentrations in the blood itself, for which the inventors sought a solution.

### SUMMARY

In an aspect of the invention there is provided the features listed in claims 1. In particular, a method is disclosed for inline measuring a substance of interest in blood fluid. Blood (or serum) is circulated through a dialysis filter having a blood inlet and a blood outlet. A dialysate circuit comprises a dialysate inlet and dialysate outlet, thereby having the substance of interest permeating via the dialysis filter to the dialysate circuit. A cuvette in the dialysate circuit is provided with a detector, for measuring concentrate levels of substances of interest in said cuvette. In a first phase a first blood flow is provided through the dialysis filter, while providing a first dialysate flow in the dialysate circuit; in a second phase a second blood flow is provided through the dialysis filter, while providing a second dialysate flow in the dialysate circuit. Concentrate levels of substances of interest in the blood fluid are derived from the measured concentrate level in the dialysate.

### BRIEF DESCRIPTION OF THE DRAWINGS

Embodiments of the invention will now be described, by way of example only, with reference to the accompanying schematic drawings in which corresponding reference symbols indicate corresponding parts, and in which:
Figure 1 shows a layout for a renal dialysis system
Figure 2 shows a dialysate circuit in countercurrent with the flow of a blood circuit
Figure 3 shows a detector capable of inline measuring ion levels of a dialysate stream;
Figure 4 shows an exemplary embodiment of a method in accordance with the present invention;
Figure 5 shows a further specific embodiment;
Figure 6 shows a further enhancement of Figure 5;
Figure 7 illustrates a rising level of concentrations in the dialysate.

### DETAILED DESCRIPTION

Unless otherwise defined, all terms (including technical and scientific terms) used herein have the same meaning as commonly understood by one of ordinary skill in the art to which this disclosure belongs as read in the context of the description and drawings. It will be further understood that terms, such as those defined in commonly used dictionaries, should be interpreted as having a meaning that is consistent with their meaning in the context of the relevant art and will not be interpreted in an idealized or overly formal sense unless expressly so defined herein. In some instances, detailed descriptions of well-known devices and methods may be omitted so as not to obscure the description of the present systems and methods. Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be further understood that the terms "comprises" and/or "comprising" specify the presence of stated features but do not preclude the presence or addition of one or more other features. All publications, patent applications, patents, and other references mentioned herein are incorporated by reference in their entirety. In case of conflict, the present specification, including definitions, will control.

The term "laser entry wall" is used to denote a wall part in the cassette that in use transmits electromagnetic laser radiation of a laser source, so that the laser beam enters the cassette interior. For a typical application for laser radiation systems, such photons may have wavelengths in the NIR or IR area, in particular, wavelengths longer than 800 nm and shorter than 1400nm. However, also wavelengths in the visible area of the electromagnetic spectrum may be used, provided an excitation spot within the cassette is provided with sufficient optical energy density in order to create emission radiation in the fluid analyte. The terms "radiation" and "beam" or "light" as used herein encompass all types of high energy electromagnetic radiation, including ultraviolet (UV) radiation, visible light (VIS) and infrared (IR) radiation. The laser entry wall may be physically distinct from other cassette wall parts, for example by suitable optimization of the optical transparency. It may also be integral to the cassette, for example manufactured by moulding.

The term "detector wall part" is used to denote a wall part in the cassette that in use transmits at least spectral parts of interest generated by the laser excited plasma, located within the fluid analyte inside the cassette, but not necessarily the laser wavelength. The detector wall part is preferably provided integrally to the cassette, but may comprise locally applied coatings and optics in order to enhance its function.

The "control mechanism" may be a dedicated processor for performing in accordance with the present system or may be a general-purpose processor wherein only one of many functions operates for performing in accordance with the present system. The processor may operate utilizing a program portion, multiple program segments, or may be a hardware device utilizing a dedicated or multi-purpose integrated circuit. Any type of processor may be used such as dedicated or shared one. The processor may include micro-controllers, central processing units (CPUs), digital signal processors (DSPs), ASICs, or any other processor(s) or controller(s) such as digital optical devices, or analog electrical circuits that perform the same functions, and employ electronic techniques and architecture. The controller or processor may further comprise a memory that may be part of or operationally coupled to the controller. The memory may be any suitable type of memory where data is stored. Any medium known or developed that can store and/or transmit information suitable for use with the present systems and methods may be used as a memory. The memory may also store user preferences and/or application data accessible by the controller for configuring it to perform operational acts in accordance with the present systems and methods.

Now turning to Figure 1 a layout is depicted for a renal dialysis system 700. In the system 700 a blood circuit 701 is formed by extracting blood from a patient and guiding it through a dialysis filter 705. Various conventional elements are provided such as a heparin pump and air embolus detector. Dialysis filter couples the blood circuit 701 via dialysis filter 705 to a dialysate circuit 702.

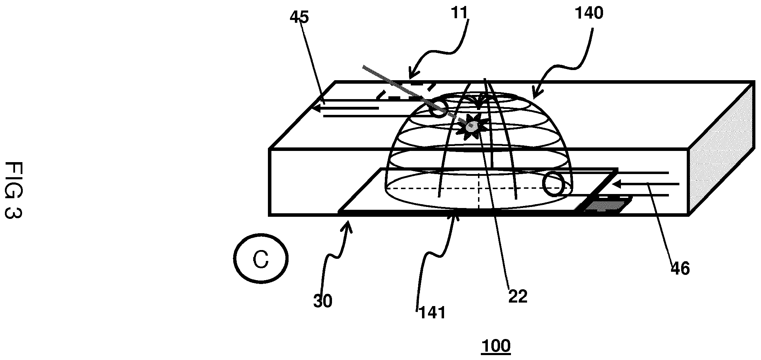

Advantageously, the dialysis filter 705 is provided with a detector cassette 710 having a photodetector 711 in the upstream part and a further photodetector 712 in the downstream part of the dialysate circuit 702. Alternatively a single detector can be utilized. The working of the detector is explained in Figure 3, but the inventive method is not dependent on a specific detector, and other suitable detectors - for example solid state single or dual grating spectrometers, mechelle spectrometers, etc. be utilized for measuring a concentrate level of said substance of interest in said cuvette in particular, of Na+, K+ and Ca2+ or other concentration levels of interest.

Advantageously, in the dialysate circuit 702 detector cassette 710 may have upstream detector 711 that measures a difference in concentration levels for predetermined concentration levels, respective to a downstream detector 712. In this way, possible saturation in ion exchange of the chemical dialysate regeneration system may be detected. For an ion mass balance the difference between the entry and exit concentration plus the fluid flow all must be known very precisely.

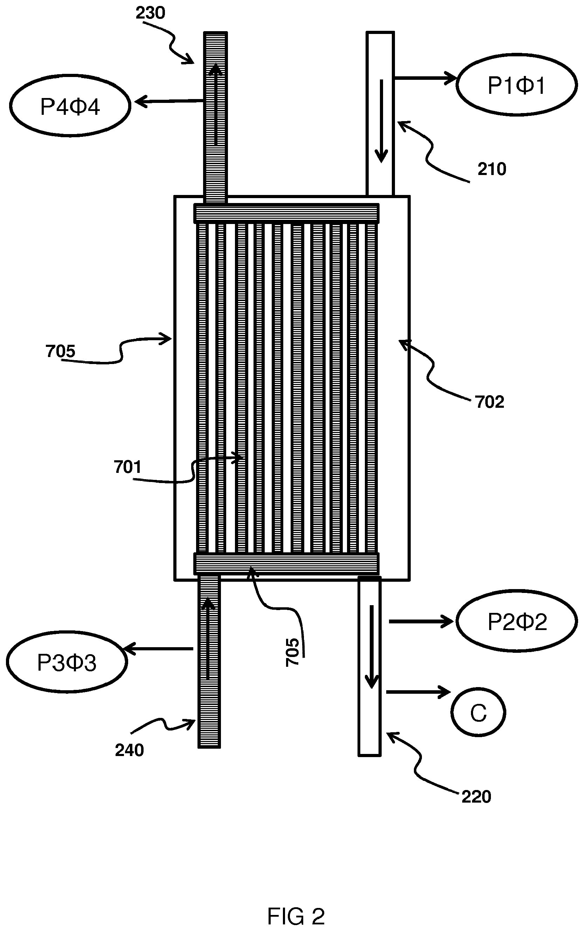

In Figure 2 it is shown that the dialysate circuit 702 is in countercurrent with the flow of blood circuit 701, separated by a membrane 703, for example, a complex of hollow semi-permeable fibres, of course also all other membranes as known within the art can be applied. The filter 705 is designed with a dialysate inlet 210 and dialysate outlet 220. The substance of interest (e.g., ions, in particular Na+, K+ and Ca2+ permeate by diffusion via the dialysis to the dialysate circuit. In the example of Figure 1, in upstream and downstream regions of the dialysate circuit (with respect to the dialysis filter 705) a detector may be provided for blood flow Φ1 through the dialysate circuit 702. Similarly, a blood flow detector for measuring flow Φ3 is provided in blood circuit 701. In the example, flow and pressure can be measured at suitable locations in either inlet 210, 240 or outlets 220, 230 of the blood circuit and dialysate circuit 702 respectively.

In Figure 3 a detector is disclosed capable of inline measuring ion levels of a dialysate stream entering at 46 and exiting at 45, by a spectroscopy device system 100. The device comprises a laser entry wall part 11 and detector wall parts 90. The laser entry wall part 11 is arranged to be optically transparent to laser radiation of predetermined a wavelength and the detector wall parts 30 are arranged to be optically transparent for spectral parts of interest to be clarified below. To avoid damage to the laser entry wall it is important that the wall is highly transparent for the laser wavelength. The laser excitation wavelength does not need to be in the same spectral range as the light emitted by the laser induced plasma.

A laser transmission optic is provided for transmitting a laser beam of the predetermined wavelength from a laser system (not shown), via the laser transmission optic to the cassette 100. The laser transmission optic is arranged to focusing the laser beam through the laser entry wall 11 in an excitation spot 22 within the cassette 100 in order to create a breakdown plasma, which emits optical radiation from within the fluid analyte. The excitation spot is a small volumetric part of the fluid analyte where the momentary laser power density is sufficiently high to create a short-living breakdown plasma discharge.

A control mechanism (not shown) is arranged to impinge the laser beam on the excitation spot within the fluid analyte, in order to create the emission radiation; and detect the emission radiation of the plasma via the detector wall 30 part by the array of photosensors in the spectral parts of interest.

A practical example is using a Nd:YaG laser at 1064nm for excitation, whilst the characteristic emissions to be detected are located at much smaller wavelengths e.g.:
Carbon (C)247.88 nm
Magnesium (Mg) 279.60, 280.30, 285.30, 516.50, 518.40, 518.90 nm
Silicon (Si) 288.20 nm
Aluminum (Al) 308.30, 309.36, 394.50, 396.20 nm
Calcium (Ca) 315.90, 318.02, 393.40, 396.86, 422.60, 442.50, 443.50, 445.50, 458.12, 458.60, 457.80, 487.80, 558.90, 559.40, 559.90, 610.30, 612.30, 616.20, 643.90, 646.30 nm
Manganese (Mn) 323.60, 325.20 nm
Iron (Fe) 373.80, 527.00 nm
Titanium multiple peaks between 428 and 432 nm, 452.70, 496.40, 504.10, 526.30, 526.60 nm
Nitrogen (N) 460.70 nm
Potassium (K) 766.49, 769.90 nm
Sodium (Na) 588.90, 589.60, 651.30 nm

The holder space in a non imaging optic is a suitably shaped non-spherical optic such as a parabolic mirror piece 140 coated with e.g. a silver coating and e.g. having a bi-convex lens 141 arranged at its end.

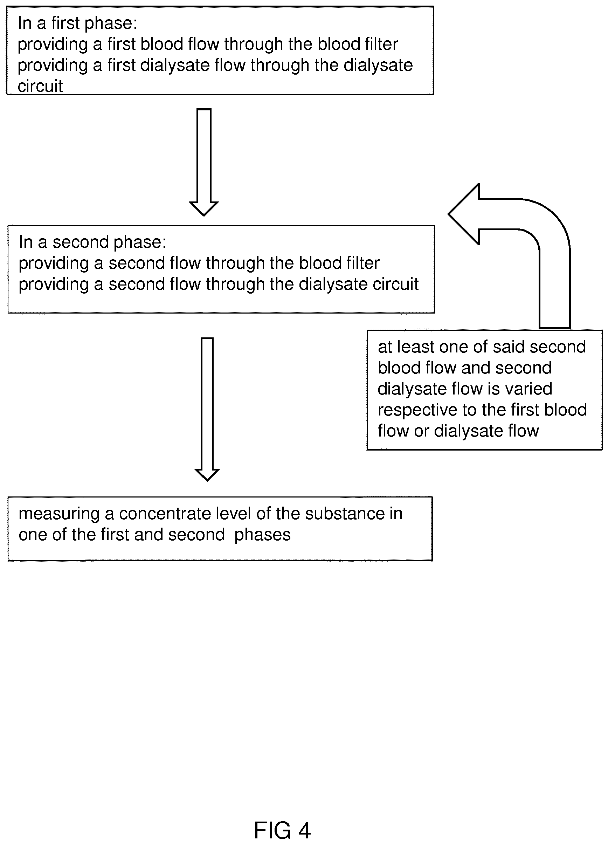

In Figure 4 an exemplary embodiment is disclosed of a method in accordance with the present invention. The method is carried out in a system for inline measurement of a substance of interest in blood fluid, comprising:
- a dialysis filter having a blood inlet and a blood outlet,
- a dialysate circuit comprising a dialysate inlet and dialysate outlet,
- a detector or pair of detectors for measuring a concentrate level in the dialysate of a substance of interest; and
- a controller programmed and arranged to carry out the following method steps.

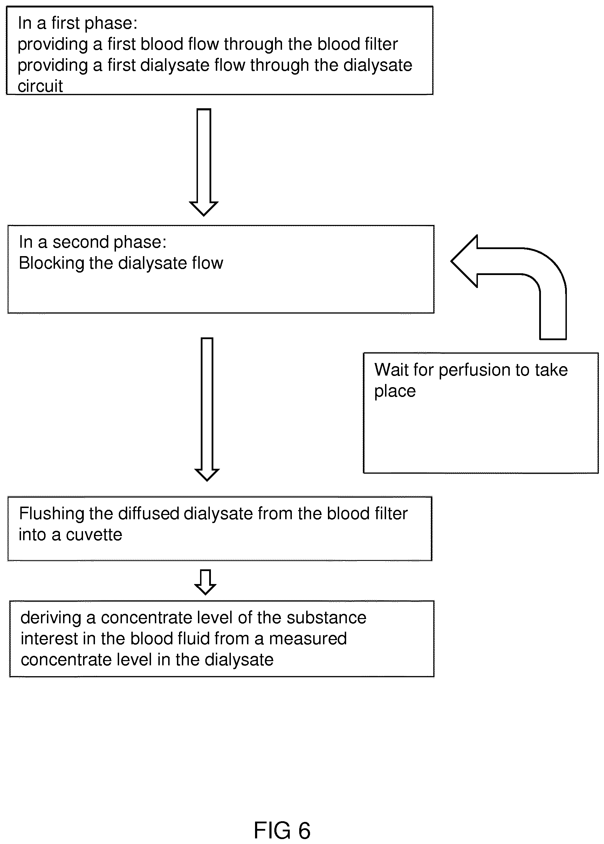

In a first phase a blood flow is provided through the dialysis filter, while providing a first dialysate flow in the dialysate circuit. This is the dialysis phase wherein dialysis is normally carried out and blood flow and dialysate flow are optimally arranged to cleanse the blood by diffusion through the dialysis filter of secretion products. In addition, ion balance is maintained in an optimal way, conventionally known, to ensure that the patient does not suffer from depletion of essential ions. For testing the substances of interest in the blood fluid, notably Na+, K+ and Ca2+ permeating by diffusion via dialysis filter. In a second phase a second blood flow is provided through the dialysis filter, while providing a second dialysate flow in the dialysate circuit. Both flows may differ from the first blood and dialysate flow respectively, and may be tuned for optimal testing purposes, as long as it is not detrimental to the patient. For example the blood pressure may be increased, by pump control, to arrive at a higher pressure in the dialysis filter, causing a higher rate known as ultrafiltration, caused by the higher convective mass transfer flux along the length of the fibers in the dialysis filter. This may enhance the perfusion of ions, shortening the time needed for the measurement. Also, for example, one or both flows may be (temporary) zero, as will be explained with reference to Figure 6.

A necessary condition is that at least one of said second blood flow and second dialysate flow is varied, e.g. by pump control in the dialysate circuit, respective to the first blood flow or dialysate flow, to provide a perfusion of the substance of interest into the dialysate different from the first phase. For this, a system controller is programmed to receive a concentrate level in the dialysate measured by said detector, of the substance in first or second phases and derive a concentrate level of the substance interest in the blood fluid from said measured concentrate level in the dialysate.

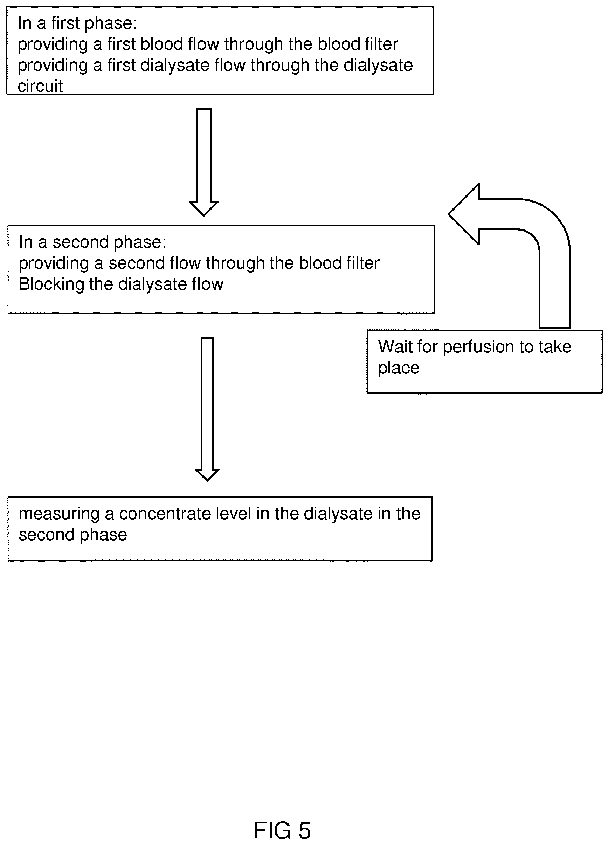

Figure 5 shows a further specific embodiment, wherein, during prior to measurement, the dialysate flow is blocked, e.g. by shutting of a pump in the dialysate circuit, thereby preventing flow of fresh dialysate into the dialysis filter. In this embodiment, a single detector suffices. A block can be carried out for example several seconds, to up to 60 seconds, depending on the perfusion constant of the dialysis filter and provided equilibrium takes place reasonably fast. In this example the blood flow is not stopped. After equilibrium between the still standing dialysate and the circulating blood is reached, the increased concentration levels in the dialysate can be measured, for example in a way described with reference to Figure 3. Note that time constants for transport of several substances can be pre-determined and be known per membrane type.

Figure 6 shows a further enhancement of Figure 5, wherein the dialysate is conveniently flushed from the dialysis filter into a detector cuvette. Although in principle, the ion levels can be measure directly in the dialysis filter (also known as dialyzer) with a suitable adaptation, it may be more convenient to flush the known volume of perfused dialysate out of the dialysis filter, into a detector cuvette, that can be situated downstream of the detector. After flushing, again the dialysate flow may be stopped, to ensure a constant composition of the substance of interest in the cuvette, and preventing it from being diluted with fresh dialysate. These steps can be carried out by the controller, controlling a pump in the dialysate circuit.

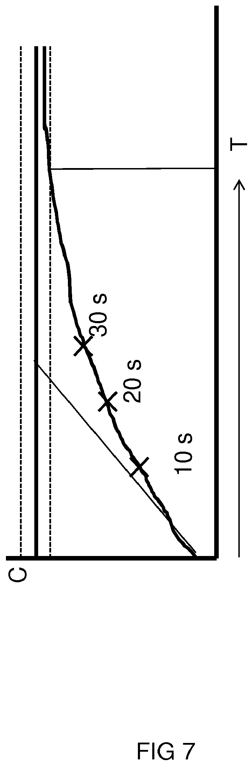

Figure 7 illustrates a rising level of concentrations in the dialysate, for example, from consecutive measurements after 10, 20 and 30 seconds. A model may be used to calculate a final state C of a concentration level, that is directly related with the substance concentration of interest in blood. Such a model can be created from empirical measurements or a diffusion calculation. E.g. wherein a diffusion constant of the dialysis filter is taken into account and the relative flow velocities between blood and dialysate streams.

While example embodiments were shown for systems and methods, also alternative ways may be envisaged by those skilled in the art having the benefit of the present disclosure for achieving a similar function and result. E.g. some components may be combined or split up into one or more alternative components. For example, the above-discussion is intended to be merely illustrative of the present system and should not be construed as limiting the appended claims to any particular embodiment or group of embodiments. Thus, while the present system has been described in particular detail with reference to specific exemplary embodiments thereof, it should also be appreciated that numerous modifications and alternative embodiments may be devised by those having ordinary skill in the art without departing from the scope of the present systems and methods as set forth in the claims that follow. The specification and drawings are accordingly to be regarded in an illustrative manner and are not intended to limit the scope of the appended claims.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. The mere fact that certain measures are recited in mutually different claims does not indicate that a combination of these measures cannot be used to advantage.

## Claims

1. A method of inline measuring a substance of interest in blood fluid, where blood fluid is circulated through a dialysis filter having a blood inlet, a blood outlet and a dialysis membrane, said dialysis filter also being connected to a dialysate circuit and further comprising a dialysate inlet and dialysate outlet, thereby having the substance of interest permeating via the dialysis membrane to the dialysate circuit, the method comprising
- providing a first blood flow through the dialysis filter in a first phase, while providing a first dialysate flow in the dialysate circuit;
- providing a second blood flow through the dialysis filter in a second phase, while providing a second dialysate flow in the dialysate circuit, wherein at least one of said second blood flow and second dialysate flow is varied respective to the first blood flow or dialysate flow, to provide an exchange of the substance of interest across the dialysis membrane;
- measuring a concentrate level in the dialysate of the substance in first or second phases and
- deriving a concentrate level of the substance interest in the blood fluid from said measured concentrate level in the dialysate.

2. A method according to claim 1, wherein said second dialysate flow is interrupted, so that said substance of interest migrates to and/or reaches equilibrium in the dialysate and subsequently measuring a concentrate level in the dialysate of the substance at zero flow of the dialysate.

3. A method according to claim 2, wherein, prior to measuring, the dialysate volume held inside the dialysis filter is flushed into a detector cuvette.

4. A method according to claim 1, wherein in said first phase a first concentrate level of said substance of interest is measured; and wherein in said second phase a second concentrate level is measured, wherein from a difference of said first and second concentrate levels a concentrate level of the substance interest in the blood fluid is derived.

5. A method according to claim 1, wherein said second blood flow is kept constant relative to the first blood flow.

6. A method according to claim 1, wherein said second blood flow is varied relative to the first blood flow, thereby varying permeation of the substance of interest through the filter.

7. A method according to any of the previous claims, further
- providing a third blood flow through the dialysis filter in a third phase, while providing a third dialysate flow in the dialysate circuit, wherein at least one of said second and third dialysate flow is varied respective to the first dialysate flow; and
- measuring a third concentrate level of the substance in the third phase and
- deriving a concentrate level of the substance interest in the blood fluid from said measured second and third concentrate level.

8. A method according to any of the previous claims, wherein a detector cuvette is provided with a laser entry wall part, and with a detector wall part that houses the detector, the laser entry wall part arranged to be optically transparent to laser radiation of predetermined a wavelength, arranged to be optically transparent for spectral parts of interest, the method further comprising
- transmitting a laser beam of the predetermined wavelength from a laser system, via a laser transmission optic to the cuvette, the laser transmission optic arranged to focussing the laser beam through the laser entry wall in an excitation spot within the cassette in order to create a laser induced plasma that emits optical radiation from within the dialysate; and
- detecting emission radiation of the plasma by a detector in spectral parts of interest.

9. A method according to claim 8, wherein the cuvette is provided downstream of the dialysis filter.

10. A system for inline measurement of a substance of interest in blood fluid, comprising:
- a dialysis filter having a blood inlet and a blood outlet,
- a dialysate circuit comprising a dialysate inlet and dialysate outlet,
- one or more detectors for measuring a concentrate level in the dialysate of a substance of interest; and
- a controller programmed and arranged to carry out the following steps
- providing a first blood flow through the dialysis filter in a first phase, while providing a first dialysate flow in the dialysate circuit;
- providing a second blood flow through the dialysis filter in a second phase, while providing a second dialysate flow in the dialysate circuit, wherein at least one of said second blood flow and second dialysate flow is varied respective to the first blood flow or dialysate flow, to provide a perfusion of the substance of interest into the dialysate;
- said controller programmed to receive a concentrate level in the dialysate measured by said detector, of the substance in first or second phases and
- programmed to derive a concentrate level of the substance interest in the blood fluid from said measured concentrate level in the dialysate.

11. The system according to claim 10, wherein said one or more detectors are integrated in respective outlets of any of the blood inlet and or dialysate inlet.
